(19) Europäisches Patentamt
European Patent Office
Office européen des brevets

(11) EP 4 485 580 A1

(12) **EUROPEAN PATENT APPLICATION**

(43) Date of publication:
**01.01.2025 Bulletin 2025/01**

(21) Application number: **24165472.2**

(22) Date of filing: **22.03.2024**

(51) International Patent Classification (IPC):
***H01M 4/62*** *(2006.01)* ***H01M 10/0567*** *(2010.01)*
***H01M 4/505*** *(2010.01)* ***H01M 4/525*** *(2010.01)*
***H01M 10/0569*** *(2010.01)*

(52) Cooperative Patent Classification (CPC):
**H01M 10/0567; H01M 4/505; H01M 4/525;**
**H01M 10/0525; H01M 10/0569;** H01M 2300/0037

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **28.06.2023 KR 20230083706**

(71) Applicant: **Samsung SDI Co., Ltd.**
**Yongin-si, Gyeonggi-do 17084 (KR)**

(72) Inventors:
- **Yang, Yeji**
**17084 Yongin-si, Gyeonggi-do (KR)**
- **Choi, Hyunbong**
**17084 Yongin-si, Gyeonggi-do (KR)**
- **Kim, Sundae**
**17084 Yongin-si, Gyeonggi-do (KR)**
- **Park, Sangwoo**
**17084 Yongin-si, Gyeonggi-do (KR)**
- **Kim, Dahyun**
**17084 Yongin-si, Gyeonggi-do (KR)**
- **Park, Hongryeol**
**17084 Yongin-si, Gyeonggi-do (KR)**
- **Kim, Sanghoon**
**17084 Yongin-si, Gyeonggi-do (KR)**

(74) Representative: **Gulde & Partner**
**Patent- und Rechtsanwaltskanzlei mbB**
**Wallstraße 58/59**
**10179 Berlin (DE)**

# (54) RECHARGEABLE LITHIUM BATTERY

(57) A rechargeable lithium battery is provided, the rechargeable lithium battery including an electrolyte solution including a non-aqueous organic solvent, a lithium salt, and an additive; positive electrode including a positive electrode active material; and a negative electrode including a negative electrode active material, wherein the non-aqueous organic solvent includes less than 5 wt% of ethylene carbonate based on a total weight of the non-aqueous organic solvent, the positive electrode active material includes lithium nickel manganese-based oxide, and the additive is represented by Chemical Formula 1.

Chemical Formula 1 is as defined in the specification.

FIG. 1

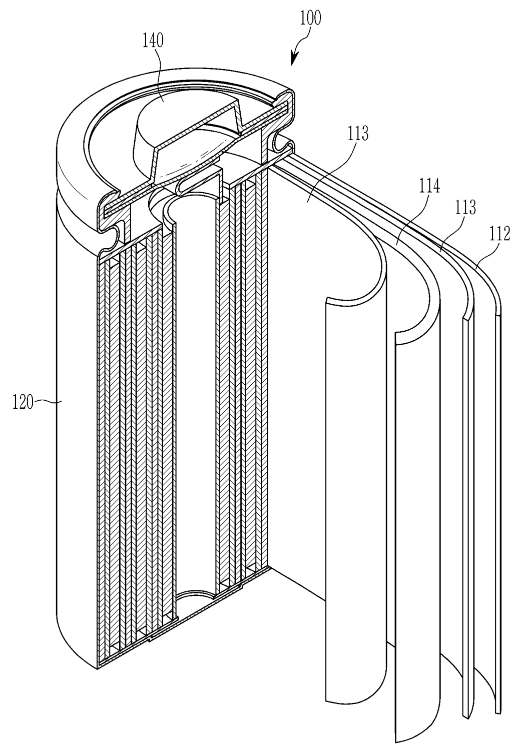


EP 4 485 580 A1

## Description

## BACKGROUND

### 1 Field

One or more embodiments of the present disclosure relate to a rechargeable lithium battery.

### 2 Description of the Related Art

A rechargeable lithium battery may be recharged and has three or more times as high (i.e., has at least three times more) energy density per unit weight than a comparable lead storage (lead-acid) battery, a comparable nickel-cadmium battery, a comparable nickel-hydrogen battery, a comparable nickel-zinc battery and/or the like. The rechargeable lithium battery may be also charged at a high rate (e.g., to a relatively high energy density) and thus, is commercially manufactured for a laptop, a cell phone, an electric tool, an electric bike, and/or the like. Researches on improvement of the rechargeable lithium battery (e.g., to provide additional energy density) have been actively made.

Furthermore, as information technology (IT) devices become increasingly more high-performance, high-capacity batteries are desired or required, for example, the high capacity may be realized by expanding a voltage region to increase energy density. However, this approach can cause complications associated with oxidizing an electrolyte solution in the high voltage region and thus deteriorating performance of a positive electrode.

Recent advances have exhibited that cobalt-free lithium nickel manganese-based oxide may be utilized as a positive electrode active material. This new material includes not cobalt, but instead nickel, manganese, and/or the like as a main component in its composition. Accordingly, a positive electrode including cobalt-free lithium nickel manganese-based oxide may be economical and realize high energy density and thus is attracting increasing attention as a next generation positive electrode active material.

However, if the positive electrode including the cobalt-free lithium nickel manganese-based oxide is utilized in a high voltage environment, transition metals may be eluted due to structural collapse of the positive electrode. This, in turn, may cause gas generation inside a cell, capacity reduction, and/or the like. Transition metal elution tends to be aggravated in a high temperature environment, wherein the eluted transition metals are precipitated on the surface of a negative electrode and may cause side reaction(s). As a result, increases in battery resistance and deterioration of battery cycle-life and output characteristics may occur.

Implementation of a positive electrode including the cobalt-free lithium nickel manganese-based oxide therefore requires (or there is a desire for) an electrolyte solution applicable under high voltage and high temperature conditions.

## SUMMARY

The invention is defined by the appended claims. The description that follows is subjected to this limitation. Any disclosure lying outside the scope of said claims is only intended for illustrative as well as comparative purposes. Aspects of example embodiments are directed toward a rechargeable lithium battery that combines a positive electrode including cobalt-free lithium nickel manganese-based oxide with an electrolyte solution capable of effectively protecting the positive electrode (i.e., the cobalt-free lithium nickel manganese-based oxide). The rechargeable lithium battery exhibits improved high-voltage characteristics and high-temperature characteristics by reducing elution of transition metals under high voltage and high temperature conditions and may thus suppress or reduce structural collapse of the positive electrode.

There is provided a rechargeable lithium battery including an electrolyte solution including a non-aqueous organic solvent, a lithium salt, and an additive; a positive electrode including a positive electrode active material; and a negative electrode including a negative electrode active material,

wherein the non-aqueous organic solvent includes less than 5 wt% of ethylene carbonate based on a total weight of the non-aqueous organic solvent,
the positive electrode active material includes a lithium nickel manganese-based oxide, and
the additive includes a compound represented by Chemical Formula 1.

Chemical Formula 1

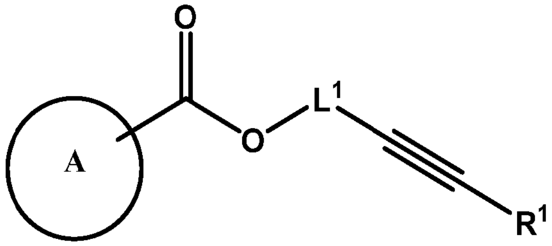

In Chemical Formula 1,

ring A is a substituted or unsubstituted C3 to C6 heteroaryl group including at least one nitrogen atom or a substituted or unsubstituted C3 to C6 heterocyclic group including at least one nitrogen atom,
$L^1$ is a substituted or unsubstituted C1 to C3 alkylene group, and
$R^1$ is hydrogen or a substituted or unsubstituted C1 to C10 alkyl group.

Some example embodiments include (e.g., may realize) a rechargeable lithium battery exhibiting improved battery stability and cycle-life characteristics by combining a positive electrode including cobalt-free lithium nickel manganese-based oxide with an electrolyte solution capable of effectively protecting the positive electrode. In some embodiments, the protection may be to secure phase transition safety of the positive electrode in a high temperature, high voltage environment and/or to suppress or reduce decomposition of the electrolyte solution and/or a side reaction with electrodes. In some embodiments, the protection may reduce gas generation and concurrently (e.g., simultaneously), suppress or reduce an increase in battery internal resistance.

## BRIEF DESCRIPTION OF THE DRAWING

The drawing is a schematic view illustrating a rechargeable lithium battery according to some example embodiments of the present invention.

## DETAILED DESCRIPTION

Hereinafter, a rechargeable lithium battery according to some example embodiments of the present disclosure will be described in more detail with reference to the accompanying drawing, wherein like reference numerals refer to like elements throughout, and duplicative descriptions thereof may not be provided. In this regard, the present embodiments may be modified to have different forms and should not be construed as being limited to the descriptions set forth herein.
As utilized herein, the term "and/or" includes any and all combinations of one or more of the associated listed items. Expressions such as "at least one of," if preceding a list of elements, modify the entire list of elements and do not modify the individual elements of the list.
If it is described that an element is "on," "connected to," or "coupled to" another element, it will be understood that the element may be provided directly on another element or still another element may be interposed therebetween. On the other hand, if it is described that an element is "directly on" another element, still another element is not interposed therebetween.
It will be understood that, although the terms "first," "second," and "third" may be utilized herein to describe one or more suitable elements, components, regions, layers, and/or sections, these elements, components, regions, layers, and/or sections should not be limited by these terms. These terms are only utilized to distinguish one element, component, region, layer, or section from another element component, region, layer, or section. Thus, a first element, component, region, layer, or section described herein may be termed a second element, component, region, layer, or section without departing from the teachings of the present specification.
As utilized herein, the singular forms "a," "an," and "the" should not be construed as being limited to the singular, but are intended to include the plural forms, including "at least one," unless the content clearly indicates otherwise. As used herein, expressions such as "at least one," "one of," and "selected from," if preceding a list of elements, may modify the entire list of elements and do not modify the individual elements of the list. For example, the expressions "at least one of a to c," "at least one of a, b and c," and "at least one of a, b and/or c" may indicate only a, only b, only c, both (e.g., simultaneously) a and b, both (e.g., simultaneously) a and c, both (e.g., simultaneously) b and c, all of a, b, and c, or variations thereof.
As utilized herein, the term "and/or" includes any and all combinations of one or more of the associated listed items. The terms "include," "includes," "including," "comprise," "comprises," "comprising," "having," "has," and/or "have" if

utilized in the detailed description, specify a presence of stated features, regions, integers, steps, operations, elements, and/or components, but do not preclude the presence or addition of one or more other features, regions, integers, steps, operations, elements, components, and/or groups thereof.

Spatially relative terms such as "beneath," "below," "lower," "above," and "upper" may be utilized herein to easily describe one element or feature’s relationship to another element or feature. It will be understood that the spatially relative terms are intended to encompass different orientations of a device in utilization or operation in addition to the orientation illustrated in the drawing. For example, if a device in the drawings is turned over, elements described as "below" or "beneath" other elements or features would then be "above" or "over" the other elements or features. Thus, the example term "below" may encompass both (e.g., opposite) orientations of above and below. The device may be otherwise oriented (rotated 90 degrees or at other orientations), and the spatially relative terms utilized herein may be interpreted accordingly.

As used herein, the terms "use," "using," and "used" may be considered synonymous with the terms "utilize," "utilizing," and "utilized," respectively.

The term "may" will be understood to refer to "one or more embodiments of the present disclosure," some of which include the described element and some of which exclude that element and/or include an alternate element. Similarly, alternative language such as "or" refers to "one or more embodiments of the present disclosure," each including a corresponding listed item.

The term "combination(s) thereof" may include a mixture, a laminate, a complex, a copolymer, an alloy, a blend, a reactant of constituents, and/or the like.

Unless otherwise defined, all terms (including chemical, technical and scientific terms) utilized herein have the same meaning as commonly understood by one of ordinary skill in the art to which the present disclosure belongs. In some embodiments, it will be further understood that terms, such as those defined in commonly utilized dictionaries, should be interpreted as having a meaning that is consistent with their meaning in the context of the relevant art and the present disclosure, and will not be interpreted in an idealized or overly formal sense unless expressly so defined herein.

In this context, "consisting essentially of" means that any additional components will not materially affect the chemical, physical, optical or electrical properties of the semiconductor film.

Further, in this specification, the phrase "on a plane," or "plan view," means viewing a target portion from the top, and the phrase "on a cross-section" means viewing a cross-section formed by vertically cutting a target portion from the side.

## Definitions

As utilized herein, the term "particle diameter" of particles refers to an average diameter if particles are spherical and refers to an average major axis length if particles are non-spherical. A particle diameter of particles may be measured utilizing a particle size analyzer (PSA). A "particle diameter" of particles is, for example, an "average particle diameter." An average particle diameter refers to, for example, a median particle diameter (D50). The median particle diameter (D50) is a particle size corresponding to a 50 % cumulative volume if a particle size distribution measured through a laser diffraction method is calculated from particles having a smaller particle size. In some embodiments, a "particle diameter" or an "average particle diameter" may be measured from a transmission electron microscope (TEM) image, a scanning electron microscope (SEM) image, and/or the like.

D50 refers to a particle size corresponding to a 50 % cumulative volume if a particle size distribution measured through a laser diffraction method is calculated from particles having a smaller particle size.

D90 refers to a particle size corresponding to a 90 % cumulative volume if a particle size distribution measured through a laser diffraction method is calculated from particles having a smaller particle size.

D10 refers to a particle size corresponding to a 10 % cumulative volume if a particle size distribution measured through a laser diffraction method is calculated from particles having a smaller particle size.

The term "positive electrode active material" as utilized herein refers to an electrode material that may undergo lithiation and delithiation.

The term "negative electrode active material" as utilized herein refers to a negative electrode material that may undergo lithiation and delithiation.

The terms "lithiate" and "lithiating" as utilized herein refer to a process of adding lithium to an electrode active material.

The terms "delithiate" and "delithiating" as utilized herein refer to a process of removing lithium from an electrode active material.

The terms "charge" and "charging" as utilized herein refer to a process of providing electrochemical energy to a battery.

The terms "discharge" and "discharging" as utilized herein refer to a process of removing electrochemical energy from a battery.

The term "positive electrode" as utilized herein refers to an electrode at which electrochemical reduction and

lithiation occur during a discharging process.

The term "negative electrode" as utilized herein refers to an electrode at which electrochemical oxidation and delithiation occur during a discharging process.

As utilized herein, if specific definition is not otherwise provided, "substituted" refers to replacement of at least one hydrogen of a substituent or a compound by deuterium, a halogen, a hydroxyl group, an amino group, a substituted or unsubstituted C1 to C30 amine group, a nitro group, a substituted or unsubstituted C1 to C40 silyl group, a C1 to C30 alkyl group, a C1 to C10 alkylsilyl group, a C6 to C30 arylsilyl group, a C3 to C30 cycloalkyl group, a C3 to C30 heterocycloalkyl group, a C6 to C30 aryl group, a C2 to C30 heteroaryl group, a C1 to C20 alkoxy group, a C1 to C10 fluoroalkyl group, a cyano group, or one or more combinations thereof.

In one example of the present disclosure, "substituted" refers to replacement of at least one hydrogen of a substituent or a compound by deuterium, a halogen, a C1 to C30 alkyl group, a C1 to C10 alkylsilyl group, a C6 to C30 arylsilyl group, a C3 to C30 cycloalkyl group, a C3 to C30 heterocycloalkyl group, a C6 to C30 aryl group, a C2 to C30 heteroaryl group, a C1 to C10 fluoroalkyl group, or a cyano group. In some embodiments, in a specific example of the present disclosure, "substituted" refers to replacement of at least one hydrogen of a substituent or a compound by deuterium, a halogen, a C1 to C20 alkyl group, a C6 to C30 aryl group, a C1 to C10 fluoroalkyl group, or a cyano group. In some embodiments, in a specific example of the present disclosure, "substituted" refers to replacement of at least one hydrogen of a substituent or a compound by deuterium, a halogen, a C1 to C5 alkyl group, a C6 to C18 aryl group, a C1 to C5 fluoroalkyl group, or a cyano group. In some embodiments, in a specific example of the present disclosure, "substituted" refers to replacement of at least one hydrogen of a substituent or a compound by deuterium, a cyano group, a halogen, a methyl group, an ethyl group, a propyl group, a butyl group, a phenyl group, a biphenyl group, a terphenyl group, a trifluoromethyl group, or a naphthyl group.

A rechargeable lithium battery may be classified into a lithium ion battery, a lithium ion polymer battery, and a lithium polymer battery depending on kinds of a separator and an electrolyte solution. It also may be classified to be cylindrical, prismatic, coin-type or kind, pouch-type or kind, and/or the like depending on shape. In some embodiments, it may be bulk type or kind and thin film type or kind depending on sizes. Structures and manufacturing methods for rechargeable lithium ion batteries pertaining to this disclosure are well suitable in the art.

Herein, as an example of a rechargeable lithium battery, a cylindrical rechargeable lithium battery is, for example, described. The drawing schematically shows the structure of a rechargeable lithium battery according to some example embodiments. Referring to the drawing, a rechargeable lithium battery 100 according to some example embodiments includes a battery cell including a positive electrode 114, a negative electrode 112 facing to the positive electrode 114, and a separator 113 between the positive electrode 114 and the negative electrode 112, and an electrolyte solution impregnating the positive electrode 114, the negative electrode 112 and the separator 113, a battery case 120 housing the battery cell, and a sealing member 140 for sealing the battery case 120.

Hereinafter, a more detailed configuration of the rechargeable lithium battery 100 according to some example embodiments is described.

**Rechargeable Lithium Battery**

A rechargeable lithium battery includes an electrolyte solution, a positive electrode, and a negative electrode.

The electrolyte solution includes a non-aqueous organic solvent, a lithium salt, and an additive, the non-aqueous organic solvent includes less than 5 wt% of ethylene carbonate based on a total weight of the non-aqueous organic solvent, and the additive includes a compound represented by Chemical Formula 1.

Chemical Formula 1

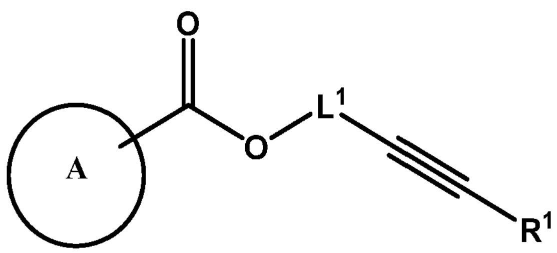

In Chemical Formula 1,

ring A is a substituted or unsubstituted C3 to C6 heteroaryl group including at least one nitrogen atom or a substituted or unsubstituted C3 to C6 heterocyclic group including at least one nitrogen atom,

$L^1$ is a substituted or unsubstituted C1 to C3 alkylene group, and

$R^1$ is hydrogen or a substituted or unsubstituted C1 to C10 alkyl group.

The positive electrode includes a positive electrode active material including lithium nickel manganese-based oxide.

In the case of positive electrode active materials including lithium nickel manganese-based oxide, e.g., cobalt-free lithium nickel manganese-based oxide, structural instability may be prevalent (e.g., is strong) under high voltage conditions, leading to solvent decomposition and elution of transition metals, especially Ni.

This transition metal elution phenomenon causes deterioration of performance of a positive electrode and short-circuiting caused by elution of transition metals, which decreases cycle-life characteristics of the battery and causes a rapid increase in resistance.

However, if the disclosed electrolyte solution is utilized together, the decrease in cycle-life characteristics of the battery and rapid increase in resistance can be alleviated.

In some embodiments, by utilizing a positive electrode including cobalt-free lithium nickel manganese-based oxide in an electrolyte solution containing less than 5 wt% of ethylene carbonate based on a total weight of the non-aqueous organic solvent, transition metal elution may be (e.g., effectively) reduced under high voltage and high temperature conditions. In some embodiments, utilizing the positive electrode and electrolyte solution disclosed herein results in suppressing the collapse of the positive electrode structure, and improving high-voltage characteristics and high-temperature characteristics of the battery.

The non-aqueous organic solvent may be (e.g., serves as) a medium for transmitting ions taking part in the electrochemical reaction of a battery.

The non-aqueous organic solvent may be a carbonate-based, ester-based, ether-based, ketone-based, alcohol-based, or aprotic solvent.

The carbonate-based solvent may include dimethyl carbonate (DMC), diethyl carbonate (DEC), dipropyl carbonate (DPC), methylpropyl carbonate (MPC), ethylpropyl carbonate (EPC), ethylmethyl carbonate (EMC), ethylene carbonate (EC), propylene carbonate (PC), butylene carbonate (BC), and/or the like. The ester-based solvent may include methyl acetate, ethyl acetate, n-propyl acetate, t-butyl acetate, methylpropionate, ethylpropionate, propylpropionate, decanolide, mevalonolactone, caprolactone, and/or the like. The ether-based solvent may include dibutyl ether, tetraglyme, diglyme, dimethoxyethane, 2-methyltetrahydrofuran, tetrahydrofuran, and/or the like. In some embodiments, the ketone-based solvent may include cyclohexanone, and/or the like. The alcohol-based solvent may include ethanol, isopropyl alcohol, and/or the like and the aprotic solvent may include nitriles such as R-CN (wherein R is a hydrocarbon group having a C2 to C20 linear, branched, or cyclic structure and may include a double bond, an aromatic ring, or an ether bond), and/or the like, dioxolanes such as 1 ,3-dioxolane, and/or the like, sulfolanes, and/or the like.

The non-aqueous organic solvent may be utilized alone or in combination with one or more of them. For example, if utilized in combination with one or more, a mixing ratio may be appropriately adjusted according to the desired or suitable battery performance, which is well understood by those skilled in the art.

The non-aqueous organic solvent includes less than 5 wt% of ethylene carbonate based on a total weight of the non-aqueous organic solvent.

If the content of ethylene carbonate is more than 5 wt% based on a total weight of the non-aqueous organic solvent, activity of Ni increases during high voltage operation, an oxidation state of Ni has a strong tendency to be reduced from tetravalent to divalent. Thus, ethylene carbonate, which has low oxidation stability, may undergo oxidative decomposition, resulting in Ni being eluted and precipitated on the negative electrode.

For example, the non-aqueous organic solvent may be a chain carbonate solvent (e.g., may be composed of a chain carbonate alone). In this case, excellent or suitable storage characteristics at a high temperature may be realized as a resistance increase rate is significantly reduced during high-temperature storage.

In the present disclosure, the meaning "composed of the chain carbonate alone" indicates that the non-aqueous organic solvent is not mixed with another non-chain carbonate solvent (e.g., cyclic carbonate and/or the like) and the non-aqueous organic solvent includes a non-aqueous organic solvent belonging to the category of the chain carbonate solvent (e.g., the non-aqueous organic solvent including one or more chain carbonates).

In some example embodiments, the chain carbonate solvent may be represented by Chemical Formula 2.

Chemical Formula 2

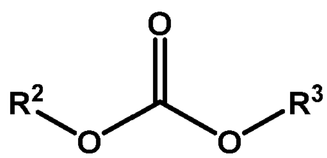

In Chemical Formula 2,

$R^2$ and $R^3$ are each independently a substituted or unsubstituted C1 to C20 alkyl group.

For example, $R^2$ and $R^3$ in Chemical Formula 2 may each independently be a substituted or unsubstituted C1 to C10 alkyl group, and for example the $R^2$ and $R^3$ may each independently be a substituted or unsubstituted C1 to C5 alkyl group.

In some example embodiments, $R^2$ and $R^3$ in Chemical Formula 2 may each independently be a substituted or unsubstituted methyl group, a substituted or unsubstituted ethyl group, a substituted or unsubstituted n-propyl group, a substituted or unsubstituted n-butyl group, a substituted or unsubstituted n-pentyl group, a substituted or unsubstituted iso-butyl group, or a substituted or unsubstituted neo-pentyl group.

For example, the non-aqueous organic solvent according to a specific embodiment may be at least two selected from among dimethyl carbonate (DMC), diethyl carbonate (DEC), dipropyl carbonate (DPC), methylpropyl carbonate (MPC), ethylpropyl carbonate (EPC), and ethylmethyl carbonate (EMC).

The non-aqueous organic solvent according to some embodiments may be a mixed solvent of dimethyl carbonate (DMC) and ethylmethyl carbonate (EMC).

The non-aqueous organic solvent may include ethylmethyl carbonate (EMC) and dimethyl carbonate (DMC) in a volume ratio of 0:100 to 50:50.

In terms of improving battery characteristics, it may be more desirable for the non-aqueous organic solvent to include more than 50 volume% of dimethyl carbonate (DMC) based on a total weight of the non-aqueous organic solvent.

For example, the non-aqueous organic solvent may include ethylmethyl carbonate (EMC) and dimethyl carbonate (DMC) in a volume ratio of 0:100 to 40:60, 0:100 to 30:70, 10:90 to 40:60, or 10:90 to 30 : 70.

The non-aqueous organic solvent may further include an aromatic hydrocarbon-based organic solvent in addition to the chain carbonate-based solvent. Herein, the chain carbonate-based solvent and the aromatic hydrocarbon-based organic solvent may be mixed in a volume ratio of 1:1 to 30:1.

The aromatic hydrocarbon-based organic solvent may be an aromatic hydrocarbon-based compound represented by Chemical Formula 3.

Chemical Formula 3

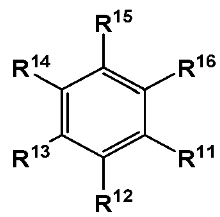

In Chemical Formula 3, $R^{11}$ to $R^{16}$ may each independently be the same or different and are selected from hydrogen, a halogen, a C1 to C10 alkyl group, a C1 to C10 haloalkyl group, and one or more combinations thereof.

Specific examples of the aromatic hydrocarbon-based organic solvent may be selected from among benzene, fluorobenzene, 1,2-difluorobenzene, 1,3-difluorobenzene, 1,4-difluorobenzene, 1,2,3-trifluorobenzene, 1,2,4-trifluorobenzene, chlorobenzene, 1,2-dichlorobenzene, 1,3-dichlorobenzene, 1,4-dichlorobenzene, 1,2,3-trichlorobenzene, 1,2,4-trichlorobenzene, iodobenzene, 1,2-diiodobenzene, 1,3-diiodobenzene, 1,4-diiodobenzene, 1,2,3-triiodobenzene, 1,2,4-triiodobenzene, toluene, fluorotoluene, 2,3-difluorotoluene, 2,4-difluorotoluene, 2,5-difluorotoluene, 2,3,4-trifluorotoluene, 2,3,5-trifluorotoluene, chlorotoluene, 2,3-dichlorotoluene, 2,4-dichlorotoluene, 2,5-dichlorotoluene, 2,3,4-trichlorotoluene, 2,3,5-trichlorotoluene, iodotoluene, 2,3-diiodotoluene, 2,4-diiodotoluene, 2,5-diiodotoluene, 2,3,4-triiodotoluene, 2,3,5-triiodotoluene, xylene, and one or more combinations thereof.

The lithium salt dissolved in the non-aqueous organic solvent supplies lithium ions in a battery, enables a general (e.g., basic) operation of a rechargeable lithium battery, and improves transportation of the lithium ions between positive and negative electrodes. Examples of the lithium salt may include at least one selected from among $LiPF_6$, $LiBF_4$, lithium difluoro(oxalato)borate (LiDFOB), $LiPO_2F_2$, $LiSbF_6$, $LiAsF_6$, $Li(FSO_2)_2N$ (lithium bis(fluorosulfonyl)imide: LiFSI), $LiC_4F_9SO_3$, $LiClO_4$, $LiAlO_2$, $LiAlCl_4$, $LiN(C_xF_{2x+1}SO_2)(C_yF_{2y+1}SO_2)$, wherein, x and y are an integer of 1 to 20, LiCl, LiI, and $LiB(C_2O_4)_2$ (lithium bis(oxalato) borate: LiBOB).

A concentration of the lithium salt may be in the range of 0.1 M to 2.0 M. If the lithium salt is included at the disclosed concentration range, an electrolyte may have excellent or suitable performance and lithium ion mobility due to optimal or suitable electrolyte conductivity and viscosity.

Ring A of Chemical Formula 1 may be a substituted or unsubstituted imidazolyl group, a substituted or unsubstituted pyrrolyl group, a substituted or unsubstituted pyrazolyl group, a substituted or unsubstituted triazolyl group, a substituted or unsubstituted pyrrolidinyl group, a substituted or unsubstituted pyridyl group, a substituted or unsubstituted pyrimidinyl group, a substituted or unsubstituted pyrazinyl group, a substituted or unsubstituted triazinyl

group, a substituted or unsubstituted piperidinyl group, or a substituted or unsubstituted piperazinyl group.

Ring A of Chemical Formula 1 may include at least one double bond.

Chemical Formula 1 may be represented by any one of Chemical Formula 1A to Chemical Formula 1O.

Chemical Formula 1A

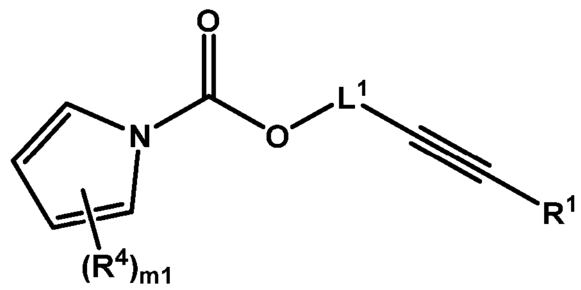

Chemical Formula 1B

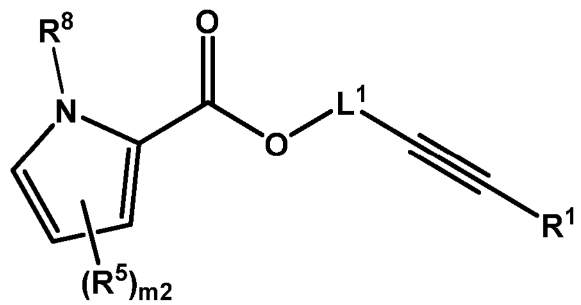

Chemical Formula 1C

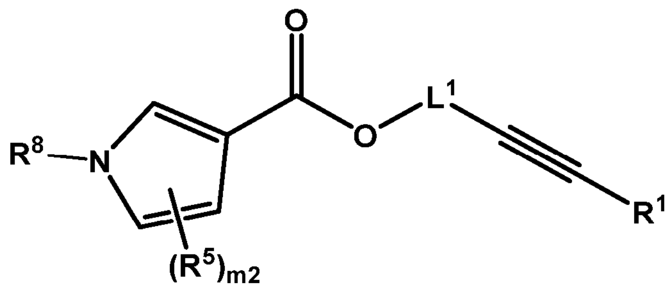

Chemical Formula 1D

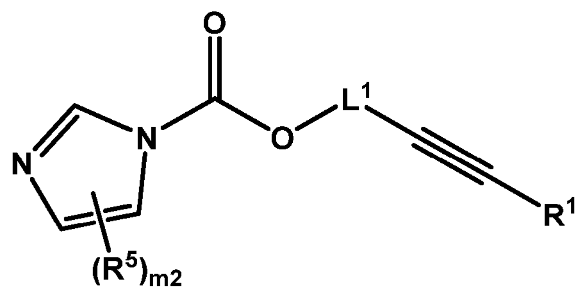

Chemical Formula 1E

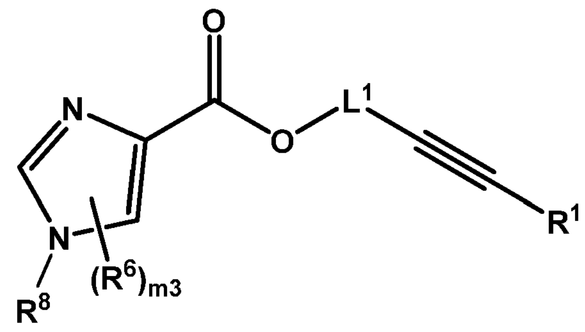

Chemical Formula 1F

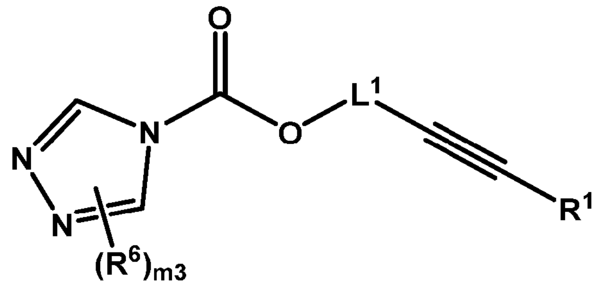

Chemical Formula 1G

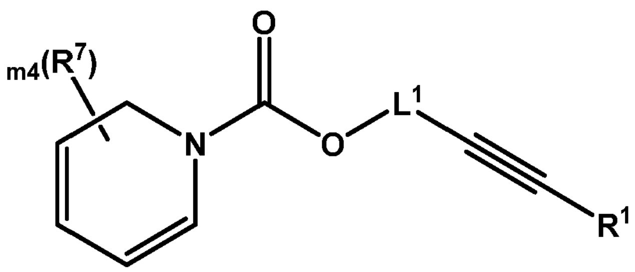

Chemical Formula 1H

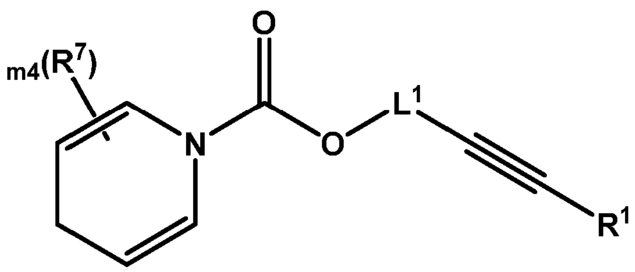

Chemical Formula 1I

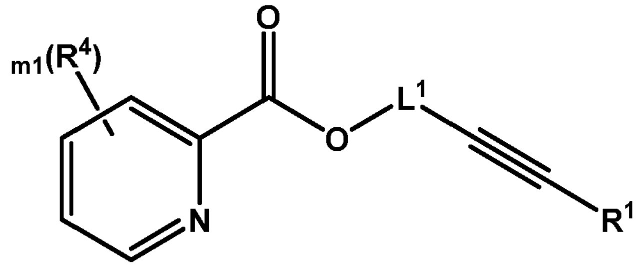

Chemical Formula 1J

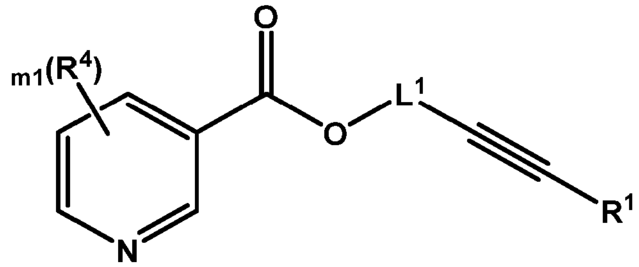

Chemical Formula 1K

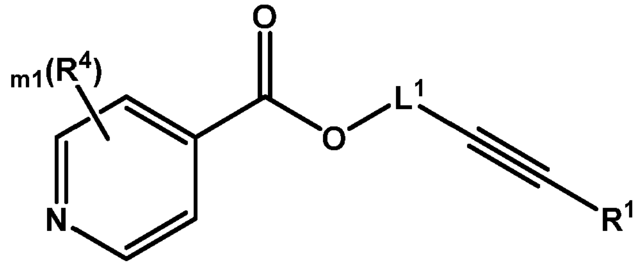

Chemical Formula 1L

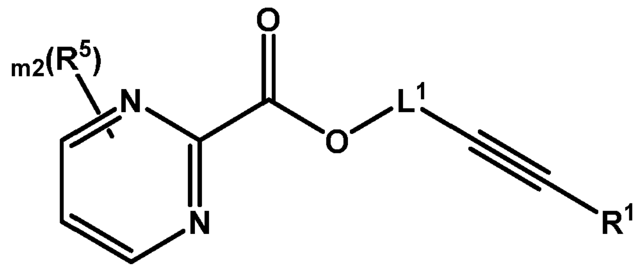

Chemical Formula 1M

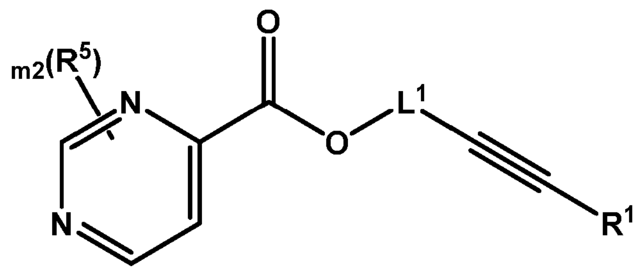

Chemical Formula 1N

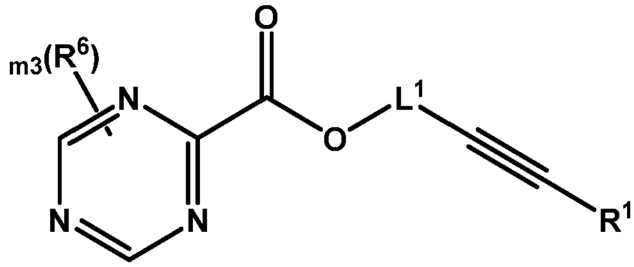

Chemical Formula 1O

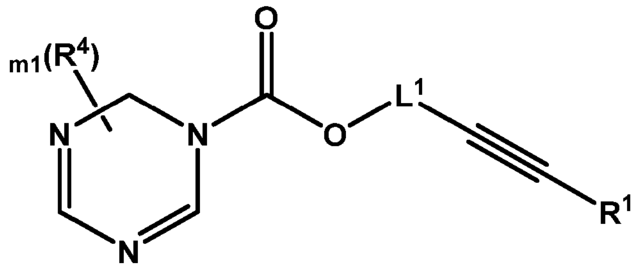

In Chemical Formula 1A to Chemical Formula 1O,

$L^1$ may be a substituted or unsubstituted C1 to C3 alkylene group,
$R^1$ and $R^4$ to $R^8$ may each independently be hydrogen or a substituted or unsubstituted C1 to C10 alkyl group,
m1 may be one of integers 1 to 4,
m2 may be one of integers 1 to 3,
m3 may be an integer of 1 or 2, and
m4 may be one of integers 1 to 6.

The compound represented by Chemical Formula 1 may be selected from compounds listed in Group 1.

Group 1

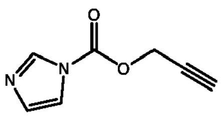
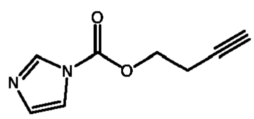
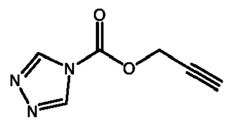
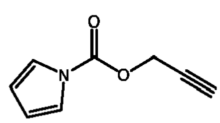

The additive may be included in an amount of 0.05 to 5.0 parts by weight based on 100 parts by weight based on 100 parts by weight of the total electrolyte solution for a rechargeable lithium battery (lithium salt + non-aqueous organic solvent) except the additive.

For example, the additive may be included in an amount of 0.05 to 3.0 parts by weight based on 100 parts by weight based on 100 parts by weight of the total electrolyte solution for a rechargeable lithium battery (lithium salt + non-aqueous organic solvent) except the additive.

For example, the additive may be included in an amount of 0.1 to 3.0 parts by weight, 0.3 to 3.0 parts by weight, 0.5 to 3.0 parts by weight, or 0.5 to 2.0 parts by weight, based on 100 parts by weight of the electrolyte solution for a rechargeable lithium battery.

If the content range of the additive is as described, an increase in resistance at high temperatures may be prevented or reduced, and a rechargeable lithium battery with improved cycle-life characteristics and output characteristics may be implemented.

On the other hand, the electrolyte solution may further include at least one of other additives selected from among vinylene carbonate (VC), fluoroethylene carbonate (FEC), difluoroethylene carbonate, chloroethylene carbonate, dichloroethylene carbonate, bromoethylene carbonate, dibromoethylene carbonate, nitroethylene carbonate, cyanoethylene carbonate, vinylethylene carbonate (VEC), adiponitrile (AN), succinonitrile (SN), 1,3,6-hexane tricyanide (HTCN), propensultone (PST), propanesultone (PS), lithium tetrafluoroborate ($LiBF_4$), lithium difluorophosphate ($LiPO_2F_2$), and 2-fluorobiphenyl (2-FBP).

By further including the disclosed other additives, the cycle-life may be further improved or gases generated from the positive electrode and the negative electrode may be effectively controlled or selected during high-temperature storage.

The other additives may be included in an amount of 0.2 to 20 parts by weight, specifically 0.2 to 15 parts by weight, or for example, 0.2 to 10 parts by weight, based on 100 parts by weight of the electrolyte solution for a rechargeable lithium battery.

If the amount of other additives is as described above, the increase in film resistance may be minimized or reduced, thereby contributing to the improvement of battery performance.

The positive electrode includes a positive electrode current collector and a positive electrode active material layer formed on the positive electrode current collector, and the positive electrode active material layer includes a positive electrode active material.

The positive electrode active material may include lithium nickel manganese-based oxide.

As an example, the lithium nickel manganese-based oxide may include at least one type or kind of a cobalt-free lithium composite oxide represented by Chemical Formula 4.

Chemical Formula 4 $Li_aNi_xMn_yM^1_zM^2_wO_{2\pm b}X_c$

In Chemical Formula 4,

$0.5 \le a < 1.8$, $0 \le b \le 0.1$, $0 \le c \le 0.1$, $0 \le w < 0.1$, $0.6 \le x < 1.0$, $0 < y < 0.4$, $0 < z < 0.1$, $w + x + y + z = 1$,

$M^1$ and $M^2$ may each independently be at least one element selected from among Al, Mg, Ti, Zr, Cr, Sr, V, B, W, Mo, Si, Ba, Ca, Ce, Fe, and Nb, and X is at least one element selected from among S, F, P, and Cl.

In the present disclosure, the cobalt-free lithium nickel manganese-based oxide as a positive electrode active material refers to a positive electrode active material composed mainly of nickel, manganese, and/or the like without including cobalt in the composition of the positive electrode active material.

The lithium composite oxides may have a coating layer on the surface, or may be mixed with another lithium composite oxide having a coating layer. The coating layer may include at least one coating element compound selected from among an oxide of a coating element, a hydroxide of a coating element, an oxyhydroxide of a coating element, an oxycarbonate of a coating element, and a hydroxy carbonate of a coating element. The compound for the coating layer may be amorphous or crystalline. The coating element included in the coating layer may include Mg, Al, Co, K, Na, Ca, Si, Ti, V, Sn, Ge, Ga, B, As, Zr, and/or a mixture thereof. The coating layer may be provided in a method having no adverse influence on properties of a positive electrode active material by utilizing these elements in the compound. For example, the method may include any coating method (e.g., spray coating, dipping, and/or the like), but is not illustrated in more detail because it is well-suitable to those skilled in the related field.

For example, Chemical Formula 4 may be represented by Chemical Formula 4-1.

Chemical Formula 4-1 $Li_aNi_{x1}Mn_{y1}Al_{z1}M^2_{w1}O_{2\pm b}X_c$

In Chemical Formula 4-1,

$0.5 \le a < 1.8$, $0 \le b \le 0.1$, $0 \le c \le 0.1$,$0 \le w1 <0.1$, $0.6 \le x1 < 1.0$, $0 < y1 <0.4$, $0 < z1 < 0.1$, $w1 + x1 + y1 + z1 = 1$,

$M^2$ is each independently at least one element selected from among Mg, Ti, Zr, Cr, Sr, V, B, W, Mo, Si, Ba, Ca, Ce, Fe, and Nb, and X is at least one element selected from among S, F, P, and Cl.

In some example embodiments, in Chemical Formula 4-1, $0.6 \le x1 \le 0.9$, $0.1 \le y1 < 0.4$, and $0 < z1 < 0.1$, or $0.6 \le x1 \le 0.8$, $0.2 \le y1 < 0.4$, and $0 < z1 < 0.1$.

For example, in Chemical Formula 4-1, x1 may be $0.6 \le x1 \le 0.79$, y1 may be $0.2 \le y1 \le 0.39$, and z1 may be $0.01 \le z1 < 0.1$.

The positive electrode active material may be included in an amount of 90 wt% to 98 wt% based on a total weight of the positive electrode active material layer.

In some example embodiments, the positive electrode active material layer may include a binder. In this case, an amount of the binder may be 1 wt% to 5 wt% based on a total weight of the positive electrode active material layer.

The binder improves binding properties of positive electrode active material particles with one another and with a positive electrode current collector. Examples thereof may be polyvinyl alcohol, carboxylmethyl cellulose, hydroxypropyl cellulose, diacetyl cellulose, polyvinylchloride, carboxylated polyvinylchloride, polyvinylfluoride, an ethylene oxide-containing polymer, polyvinylpyrrolidone, polyurethane, polytetrafluoroethylene, polyvinylidene fluoride, polyethylene, polypropylene, a styrene-butadiene rubber, an acrylated styrene-butadiene rubber, an epoxy resin, nylon, and/or the like, but is not limited thereto.

Al may be utilized as the positive electrode current collector, but is not limited thereto.

The negative electrode includes a negative electrode current collector and a negative electrode active material layer including a negative electrode active material formed on the negative electrode current collector.

The negative electrode active material may include a material that reversibly intercalates/deintercalates lithium ions, a lithium metal, a lithium metal alloy, a material capable of doping/dedoping lithium, or transition metal oxide.

The material that reversibly intercalates/deintercalates lithium ions may include a carbon material. The carbon material may be any generally-utilized carbon-based negative electrode active material in a rechargeable lithium battery. Examples thereof may be crystalline carbon, amorphous carbon, or a mixture thereof. The crystalline carbon may be non-shaped, or sheet, flake, spherical, or fiber shaped natural graphite or artificial graphite. The amorphous carbon may be a soft carbon, a hard carbon, a mesophase pitch carbonization product, calcined coke, and/or the like.

The lithium metal alloy includes an alloy of lithium and a metal selected from among Na, K, Rb, Cs, Fr, Be, Mg, Ca, Sr, Si, Sb, Pb, In, Zn, Ba, Ra, Ge, Al, and Sn.

The material capable of doping/dedoping lithium may be Si, Si-C composite, $SiO_x$ ($0 < x \leq 2$), a Si-Q alloy (wherein Q is an element selected from among an alkali metal, an alkaline-earth metal, a Group 13 element, a Group 14 element except Si, a Group 15 element, a Group 16 element, a transition metal, a rare earth element, and one or more combinations thereof), Sn, $SnO_x$ ($0 < x \leq 2$), a Sn-$R^{11}$ alloy (wherein $R^{11}$ is an element selected from among an alkali metal, an alkaline-earth metal, a Group 13 element, a Group 14 element except Sn, a Group 15 element, a Group 16 element, a transition metal, a rare earth element, and one or more combinations thereof), and/or the like. At least one of these materials may be mixed with $SiO_2$.

The elements Q and $R^{11}$ may be selected from among Mg, Ca, Sr, Ba, Ra, Sc, Y, Ti, Zr, Hf, Rf, V, Nb, Ta, Db, Cr, Mo, W, Sg, Tc, Re, Bh, Fe, Pb, Ru, Os, Hs, Rh, Ir, Pd, Pt, Cu, Ag, Au, Zn, Cd, B, Al, Ga, Sn ($R^{11}$ does not comprised Sn), In, Tl, Ge, P, As, Sb, Bi, S, Se, Te, Po, and one or more combinations thereof.

The transition metal oxide may be vanadium oxide, lithium vanadium oxide, or lithium titanium oxide.

In some example embodiments, the negative electrode active material may include at least one selected from among graphite and a Si composite.

The Si composite may include a core including Si-based particles and an amorphous carbon coating layer. For example, the Si-based particles may include (e.g., may be) at least one (e.g., one or more) selected from among silicon particles, Si-C composite, $SiO_x$ ($0 < x \leq 2$), and Si alloy.

For example, the core including the Si-based particles may include a void at the central portion, the radius of the central portion corresponds to 30% to 50% of the radius of the negative electrode active material, and the average particle diameter of the Si-based particles may be 10 nanometer (nm) to 200 nm.

In the present disclosure, an average particle diameter (D50) may be particle size at a volume ratio of 50% in a cumulative size-distribution curve.

If the Si-based particles have an average particle diameter within the range, volume expansion during the charge and discharge may be suppressed or reduced, and interruption of a conductive path due to particle crushing during the charge and discharge may be prevented or reduced.

The core including the Si-based particles further includes amorphous carbon, and, here, the central portion does not include amorphous carbon, for example, the amorphous carbon may exist in the surface portion (alone) of the negative electrode active material.

In some embodiments, the surface portion refers to an area from the outermost surface of the central portion to the outermost surface of the negative electrode active material.

In some embodiments, the Si-based particles are included substantially uniformly throughout the Si composite, and may exist in a substantially uniform concentration in the central and surface portions.

The amorphous carbon may be soft carbon, hard carbon, a mesophase pitch carbonized product, calcined coke, or one or more combinations thereof.

In one or more embodiments, the negative electrode active material may include (e.g., be) a Si-C composite, and the Si-C composite, here, may include silicon particles and crystalline carbon.

The silicon particles may be included in an amount of 1 to 60 wt%, for example, 3 to 60 wt% based on a total weight of the Si-C composite.

Examples of the crystalline carbon may include graphite, and for example may be natural graphite, artificial graphite, or a combination thereof.

An average particle diameter of the crystalline carbon may be 5 micrometer ($\mu$m) to 30 $\mu$m.

If the negative electrode active material includes both (e.g., simultaneously) graphite and the Si composite, the graphite and the Si composite may be included as (e.g., in the form of) a mixture. In this case, the graphite and the Si composite may be included in a weight ratio of 99:1 to 50:50.

In some embodiments, the graphite and the Si composite may be included in a weight ratio of 97:3 to 80:20 or 95:5 to 80:20.

The amorphous carbon precursor may include a coal-based pitch, mesophase pitch, petroleum-based pitch, coal-based oil, petroleum-based heavy oil, or a polymer resin such as a phenol resin, a furan resin, or a polyimide resin.

In the negative electrode active material layer, the negative electrode active material may be included in an amount of 95 wt% to 99 wt% based on a total weight of the negative electrode active material layer.

In some example embodiments of the present disclosure, the negative electrode active material layer further includes a binder, and optionally a conductive material. In the negative electrode active material layer, a content of the binder may be 1 wt% to 5 wt% based on a total weight of the negative electrode active material layer. If the negative electrode active material layer includes a conductive material, the negative electrode active material layer includes 90 wt% to 98 wt% of the negative electrode active material, 1 wt% to 5 wt% of the binder, and 1 wt% to 5 wt% of the conductive material.

The binder improves binding properties of negative electrode active material particles with one another and with a negative electrode current collector. The binder includes a non-water-soluble binder, a water-soluble binder, or a combination thereof.

The non-water-soluble binder may be selected from polyvinylchloride, carboxylated polyvinylchloride, polyvinylfluoride, polyurethane, polytetrafluoroethylene, polyvinylidene fluoride, polyethylene, polypropylene, polyamideimide, polyimide, or one or more combinations thereof.

The water-soluble binder may be a rubber-based binder or a polymer resin binder. The rubber-based binder may be selected from among a styrene-butadiene rubber, an acrylated styrene-butadiene rubber (SBR), an acrylonitrile-butadiene rubber, an acrylic rubber, a butyl rubber, a fluorine rubber, and one or more combinations thereof. The polymer resin binder may be selected from among polytetrafluoroethylene, ethylene propylene copolymer, polyethylene oxide, polyvinylpyrrolidone, polyepichlorohydrin, polyphosphazene, polyacrylonitrile, polystyrene, an ethylene propylene diene copolymer, polyvinylpyridine, chlorosulfonated polyethylene, latex, a polyester resin, an acrylic resin, a phenolic resin, an epoxy resin, polyvinyl alcohol, and one or more combinations thereof.

If the water-soluble binder is utilized as a binder in the negative electrode active material layer, a cellulose-based compound as a thickener may be further utilized to provide viscosity. The cellulose-based compound includes one or more of carboxymethyl cellulose, hydroxypropylmethyl cellulose, methyl cellulose, and alkali metal salts thereof. The alkali metals may be Na, K, or Li. Such a thickener may be included in an amount of 0.1 to 3 parts by weight based on 100 parts by weight of the negative electrode active material.

The conductive material is included to provide electrode conductivity and any electrically conductive material may be utilized as a conductive material unless it causes a chemical change. Examples of the conductive material include a carbon-based material such as natural graphite, artificial graphite, carbon black, acetylene black, ketjen black, a carbon fiber, and/or the like; a metal-based material of a metal powder or a metal fiber including copper, nickel, aluminum silver, and/or the like; a conductive polymer such as a polyphenylene derivative; or a mixture thereof.

The negative electrode current collector may include one selected from among a copper foil, a nickel foil, a stainless steel foil, a titanium foil, a nickel foam, a copper foam, a polymer substrate coated with a conductive metal, and one or more combinations thereof.

The rechargeable lithium battery may further include a separator between the negative electrode and the positive electrode, depending on a type or kind of the rechargeable lithium battery. Examples of a suitable separator material include at least one selected from among polyethylene, polypropylene, polyvinylidene fluoride, and multi-layers thereof such as a polyethylene/polypropylene double-layered separator, a polyethylene/polypropylene/polyethylene triple-layered separator, and a polypropylene/polyethylene/polypropylene triple-layered separator.

The rechargeable lithium battery may have an upper charge limit voltage of greater than or equal to 4.35 V. For example, the upper charge limit voltage may be 4.35 V to 4.55 V.

Numerical ranges disclosed herein include and are intended to disclose all subsumed sub-ranges of the same numerical precision. For example, a range of "1.0 to 10.0" includes all subranges having a minimum value equal to or greater than 1.0 and a maximum value equal to or less than 10.0, such as, for example, 2.4 to 7.6. Applicant therefore reserves the right to amend this specification, including the claims, to expressly recite any sub-range subsumed within the ranges expressly recited herein.

Hereinafter, examples of the present disclosure and comparative examples are described. These examples, however, are not in any sense to be interpreted as limiting the scope of the present disclosure.

## EXAMPLES

### Manufacture of Rechargeable Lithium Battery Cell

#### Example 1

$LiNi_{0.75}Mn_{0.23}Al_{0.02}O_2$ as a positive electrode active material, polyvinylidene fluoride as a binder, and acetylene black as a conductive material were mixed in a weight ratio of 96:3:1 and then, dispersed in N-methyl pyrrolidone, preparing a positive electrode active material slurry.

The positive electrode active material slurry was coated on a 15 μm-thick Al foil, dried at 100 °C, and pressed, to manufacture a positive electrode.

A negative electrode active material slurry was also prepared by utilizing a mixture of artificial graphite and Si composite in a weight ratio of 93:7 as a negative electrode active material and then, mixing the negative electrode active material, a styrene-butadiene rubber binder, and carboxylmethyl cellulose in a weight ratio of 98:1:1 and dispersing the obtained mixture in distilled water.

As for the Si composite, a core including artificial graphite and silicon particles was coated with coal-based pitch on the surface.

The negative electrode active material slurry was coated on a 10 μm-thick Cu foil, dried at 100 °C, and pressed, to manufacture a negative electrode.

The manufactured positive and negative electrodes were assembled with a 10 μm-thick polyethylene separator to manufacture an electrode assembly, and an electrolyte solution was injected thereinto, to manufacture a rechargeable lithium battery cell.

The electrolyte solution had a following composition.

**Composition of electrolyte solution**

lithium salt: 1.5 M $LiPF_6$
non-aqueous organic solvent: ethylmethyl carbonate : dimethyl carbonate (EMC:DMC = volume ratio of 20:80)
additive: 1 part by weight of a compound represented by Chemical Formula 1-1 (additive alone)

Chemical Formula 1-1

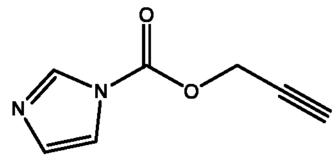

In the above composition of the electrolyte solution, "parts by weight" refers to the relative weight of the additive based on 100 parts by weight of the total electrolyte solution (lithium salt + non-aqueous organic solvent) except the additive.

**Comparative Example 1**

A rechargeable lithium battery cell was manufactured in substantially the same manner as in Example 1, except that the compound represented by Chemical Formula 1-1 in the composition of the electrolyte solution was not added.

**Comparative Example 2**

A rechargeable lithium battery cell was manufactured in substantially the same manner as in Example 1, except that 20 wt% of ethylene carbonate was added based on a total weight of the non-aqueous organic solvent in the composition of the electrolyte solution.

**Comparative Example 3**

A rechargeable lithium battery cell was manufactured in substantially the same manner as in Example 1, except that 1 part by weight of fluoroethylene carbonate (FEC) was added instead of the compound represented by Formula 1-1 in the composition of the electrolyte solution.

**Example 2**

A rechargeable lithium battery cell was manufactured in substantially the same manner as in Example 1, except that the non-aqueous organic solvent was changed to dimethyl carbonate at a volume ratio of 100, i.e., only dimethyl carbonate was used as the non-aqueous organic solvent.

**Example 3**

A rechargeable lithium battery cell was manufactured in substantially the same manner as in Example 1, except

that a mixing ratio of ethylmethyl carbonate and dimethyl carbonate was changed to a volume ratio of 40:60, respectively.

**Comparative Examples 4 to 6**

Rechargeable lithium battery cells were manufactured in substantially the same manner as Example 1 and Comparative Examples 1 and 2, respectively, except that the positive electrode active material was changed to $LiCoO_2$.

**Comparative Examples 7 to 9**

Rechargeable lithium battery cells were manufactured in substantially the same manner as Example 1 and Comparative Examples 1 and 2, respectively, except that the positive electrode active material was changed to $LiNi_{0.5}Co_{0.2}Al_{0.3}O_2$.

**Comparative Examples 10 to 12**

Rechargeable lithium battery cells were manufactured in substantially the same manner as Example 1 and Comparative Examples 1 and 2, respectively, except that the positive electrode active material was changed to $LiNi_{0.8}Co_{0.1}Mn_{0.1}O_2$.

**Evaluation 1: Evaluation of storage characteristics at high temperature**

The rechargeable lithium battery cells according to Examples 1 to 3 and Comparative Examples 1 to 12 were measured with respect to initial DC internal resistance (DCIR) as $\Delta V/\Delta I$ (voltage change/current change), then, DC internal resistance thereof was measured again by setting a maximum energy state inside the rechargeable lithium battery cells to a fully charged state (SOC 100%) and then, storing them at a high temperature of 60 °C for 30 days to calculate a DCIR increase rate (%) according to Equation 1, and the results are shown in Table 1.

Equation 1

$$\text{DCIR increase rate} = \{(\text{DCIR after 30 days}) / (\text{initial DCIR})\} \times 100$$

**Evaluation 2: Evaluation of high temperature cycle-life characteristics**

The rechargeable lithium battery cells according to Examples 1 to 3 and Comparative Examples 1 to 12 were once charged and discharged at 0.2 C and then, measured with respect to charge and discharge capacity.

In some embodiments, the rechargeable lithium battery cells manufactured according to Examples 1 to 3 and Comparative Examples 1 to 12 were charged at an upper charge limit voltage of 4.4 V, and then discharged at 0.2 C to 2.5 V under constant current conditions to measure the initial discharge capacity.

Discharge capacity was measured by performing 200 cycles of charge and discharge again under conditions of 0.33 C charge (CC/CV, 4.4 V, 0.025 C cut-off)/1.0 C discharge (CC, 2.5 V cut-off) and 45 °C. The discharge capacity ratio to the initial discharge capacity is shown as capacity recovery rate (%, recovery) in Table 2.

**Evaluation 3: Measurement of gas generation after high-temperature storage**

The rechargeable lithium battery cells according to Example 1 and Comparative Examples 1 to 12 were allowed to stand at 60 °C for 7 days, and then, refinery gas analysis (RGA) was utilized to measure gas generation amounts (mL) at the 1st day and the 7th day, the increase rates were calculated, and the results are shown in Table 3.

The increase rate was calculated according to Equation 2.

$$\text{Increase rate} = \{(\text{Gas generation amount on the 7th day})/(\text{Gas generation amount on the 1st day})\} \times 100 \qquad \text{equation 2}$$

Table 1

| | | Initial DCIR (mOhm) | DCIR after high-temperature (60 °C) storage for 30 days (mOhm) | DCIR Increase rate (60 °C, 30 days) (%) |
|---|---|---|---|---|
| $LiNi_{0.75}Mn_{0.23}Al_{0.02}O_2$ | Example 1 | 42.3 | 44.2 | 104.5 |
| | Example 2 | 44.2 | 47.0 | 106.3 |
| | Example 3 | 44.3 | 47.2 | 106.5 |
| | Comparative Example 1 | 45.4 | 48.8 | 107.6 |
| | Comparative Example 2 | 43.7 | 46.3 | 105.9 |
| | Comparative Example 3 | 43.2 | 45.5 | 105.4 |
| $LiCoO_2$ | Comparative Example 4 | 43.5 | 46.0 | 105.7 |
| | Comparative Example 5 | 43.7 | 46.3 | 105.9 |
| | Comparative Example 6 | 43.2 | 45.5 | 105.4 |
| $LiNi_{0.5}Co_{0.2}Al_{0.3}O_2$ | Comparative Example 7 | 43.3 | 45.7 | 105.5 |
| | Comparative Example 8 | 43.5 | 46.0 | 105.7 |
| | Comparative Example 9 | 43 | 45.2 | 105.2 |
| $LiNi_{0.8}Co_{0.1}Mn_{0.1}O_2$ | Comparative Example 10 | 43.4 | 45.8 | 105.6 |
| | Comparative Example 11 | 43.6 | 46.1 | 105.8 |
| | Comparative Example 12 | 43.1 | 45.4 | 105.3 |

Table 2

| | | Capacity recovery rate (45 °C, 200 cy, %) |
|---|---|---|
| $LiNi_{0.75}Mn_{0.23}Al_{0.02}O_2$ | Example 1 | 97.5 |
| | Example 2 | 95.2 |
| | Example 3 | 95.4 |
| | Comparative Example 1 | 85.1 |
| | Comparative Example 2 | 94.5 |
| | Comparative Example 3 | 95.2 |
| $LiCoO_2$ | Comparative Example 4 | 94.8 |
| | Comparative Example 5 | 93.5 |
| | Comparative Example 6 | 93.7 |
| $LiNi_{0.5}Co_{0.2}Al_{0.3}O_2$ | Comparative Example 7 | 94.9 |
| | Comparative Example 8 | 94.1 |
| | Comparative Example 9 | 92.9 |
| $LiNi_{0.8}Co_{0.1}Mn_{0.1}O_2$ | Comparative Example 10 | 94.6 |
| | Comparative Example 11 | 93.6 |
| | Comparative Example 12 | 93.5 |

Table 3

| | | Gas generation after storage at high temperature (60 °C) | | |
|---|---|---|---|---|
| | | 1st day (mL) | 7th (mL) | Increa se rate (%) |
| $LiNi_{0.75}Mn_{0.23}Al_{0.02}O_2$ | Example 1 | 1.5 | 2.1 | 140 |
| | Comparative Example 1 | 1.9 | 3.1 | 163 |
| | Comparative Example 2 | 2.1 | 3.2 | 152 |
| | Comparative Example 3 | 2.2 | 3.6 | 164 |
| $LiCoO_2$ | Comparative Example 4 | 2.4 | 3.8 | 158 |
| | Comparative Example 5 | 2.3 | 3.7 | 161 |
| | Comparative Example 6 | 2.1 | 3.5 | 167 |
| $LiNi_{0.5}Co_{0.2}Al_{0.3}O_2$ | Comparative Example 7 | 2.3 | 3.7 | 161 |
| | Comparative Example 8 | 2.4 | 3.8 | 158 |
| | Comparative Example 9 | 2.6 | 4 | 154 |
| $LiNi_{0.8}Co_{0.1}Mn_{0.1}O_2$ | Comparative Example 10 | 2.7 | 4.1 | 152 |
| | Comparative Example 11 | 2.4 | 3.8 | 158 |
| | Comparative Example 12 | 2.5 | 3.9 | 156 |

Referring to Tables 1 to 3, in the composition combining the electrolyte solution and positive electrode active material according to the present application, the DCIR increase rate is reduced, improving both (e.g., simultaneously) high-temperature storage characteristics and high-temperature charge and discharge characteristics.

In some embodiments, the amounts of gas generated in the rechargeable lithium battery cells according to the examples were significantly reduced after being stored at high temperature.

## Description of symbols

100: rechargeable lithium battery
112: negative electrode
113: separator
114: positive electrode
120: battery case
140: sealing member

## Claims

**1.** A rechargeable lithium battery (100), comprising

an electrolyte solution comprising a non-aqueous organic solvent, a lithium salt, and an additive;
a positive electrode (114) comprising a positive electrode active material; and
a negative electrode (112) comprising a negative electrode active material,
wherein the non-aqueous organic solvent comprises less than 5 wt% of ethylene carbonate based on a total weight of the non-aqueous organic solvent,
the positive electrode active material comprises a lithium nickel manganese-based oxide, and
the additive is a compound represented by Chemical Formula 1:

Chemical Formula 1

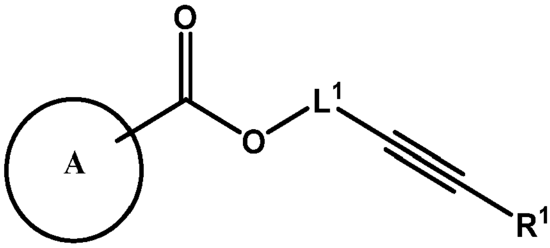

,

and

wherein, in Chemical Formula 1,

ring A is a substituted or unsubstituted C3 to C6 heteroaryl group comprising at least one nitrogen atom or a substituted or unsubstituted C3 to C6 heterocyclic group comprising at least one nitrogen atom,

$L^1$ is a substituted or unsubstituted C1 to C3 alkylene group, and

$R^1$ is hydrogen or a substituted or unsubstituted C1 to C10 alkyl group,

wherein "substituted" refers to replacement of at least one hydrogen of a substituent or a compound by deuterium, a halogen, a hydroxyl group, an amino group, a substituted or unsubstituted C1 to C30 amine group, a nitro group, a substituted or unsubstituted C1 to C40 silyl group, a C1 to C30 alkyl group, a C1 to C10 alkylsilyl group, a C6 to C30 arylsilyl group, a C3 to C30 cycloalkyl group, a C3 to C30 heterocycloalkyl group, a C6 to C30 aryl group, a C2 to C30 heteroaryl group, a C1 to C20 alkoxy group, a C1 to C10 fluoroalkyl group, and a cyano group.

**2.** The rechargeable lithium battery of claim 1, wherein the non-aqueous organic solvent is a chain carbonate solvent.

**3.** The rechargeable lithium battery of claim 2, wherein

the chain carbonate solvent is represented by Chemical Formula 2:

Chemical Formula 2

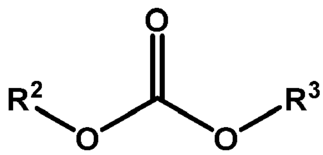

,

and

wherein, in Chemical Formula 2

$R^2$ and $R^3$ are each independently a substituted or unsubstituted C1 to C20 alkyl group,

wherein "substituted" refers to replacement of at least one hydrogen of a substituent or a compound by deuterium, a halogen, a hydroxyl group, an amino group, a substituted or unsubstituted C1 to C30 amine group, a nitro group, a substituted or unsubstituted C1 to C40 silyl group, a C1 to C30 alkyl group, a C1 to C10 alkylsilyl group, a C6 to C30 arylsilyl group, a C3 to C30 cycloalkyl group, a C3 to C30 heterocycloalkyl group, a C6 to C30 aryl group, a C2 to C30 heteroaryl group, a C1 to C20 alkoxy group, a C1 to C10 fluoroalkyl group, and a cyano group.

**4.** The rechargeable lithium battery of any one of the preceding claims, wherein the non-aqueous organic solvent is at least two selected from among dimethyl carbonate (DMC), diethyl carbonate (DEC), dipropyl carbonate (DPC), methylpropyl carbonate (MPC), ethylpropyl carbonate (EPC), and ethylmethyl carbonate (EMC).

**5.** The rechargeable lithium battery of any one of the preceding claims, wherein the non-aqueous organic solvent comprises ethylmethyl carbonate (EMC) and dimethyl carbonate (DMC) in a volume ratio of 0:100 to 50:50.

6. The rechargeable lithium battery of any one of the preceding claims, wherein ring A of Chemical Formula 1 is a substituted or unsubstituted imidazolyl group, a substituted or unsubstituted pyrrolyl group, a substituted or unsubstituted pyrazolyl group, a substituted or unsubstituted triazolyl group, a substituted or unsubstituted pyrrolidinyl group, a substituted or unsubstituted pyridyl group, a substituted or unsubstituted pyrimidinyl group, a substituted or unsubstituted pyrazinyl group, a substituted or unsubstituted triazinyl group, a substituted or unsubstituted piperidinyl group, or a substituted or unsubstituted piperazinyl group.

7. The rechargeable lithium battery of claim 1, wherein ring A of Chemical Formula 1 comprises at least one double bond.

8. The rechargeable lithium battery of claim 1, wherein

Chemical Formula 1 is represented by any one of Chemical Formula 1A to Chemical Formula 1O:

Chemical Formula 1A

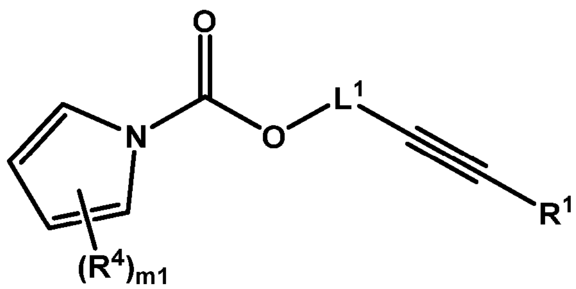

Chemical Formula 1B

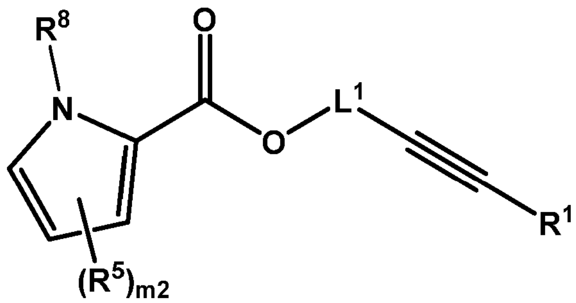

Chemical Formula 1C

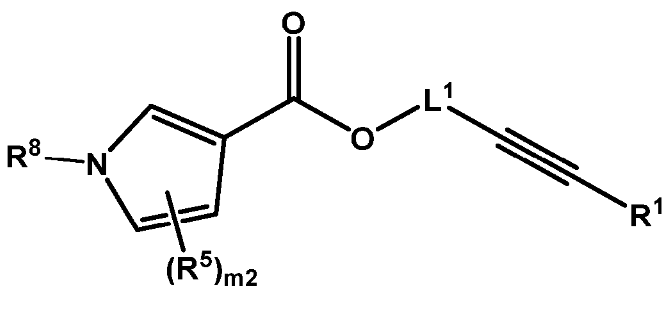

Chemical Formula 1D

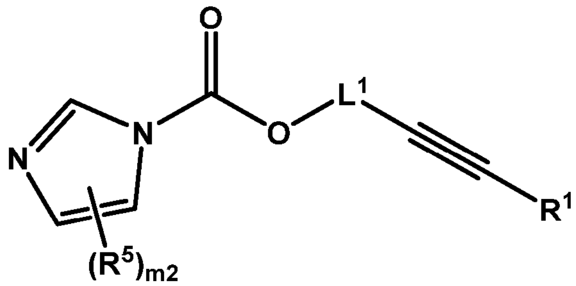

Chemical Formula 1E

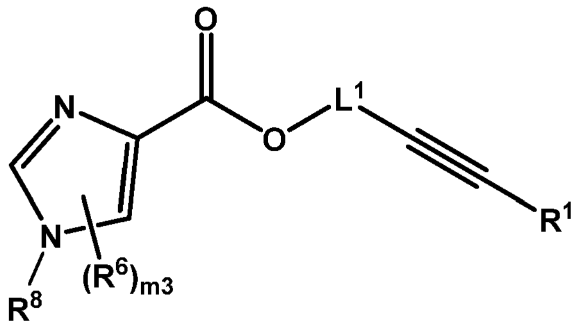

Chemical Formula 1F

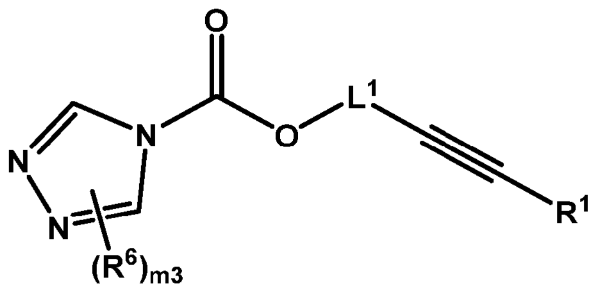

Chemical Formula 1G

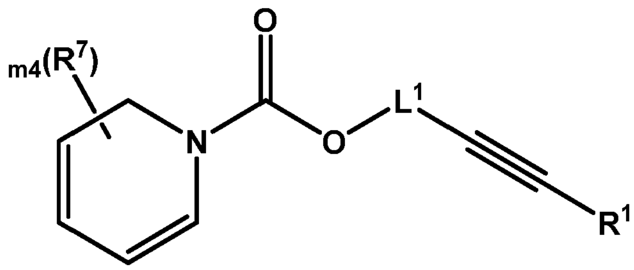

Chemical Formula 1H

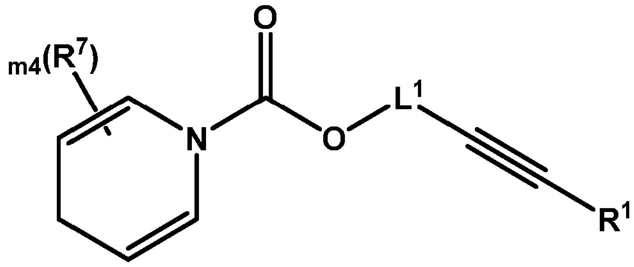

Chemical Formula 1I

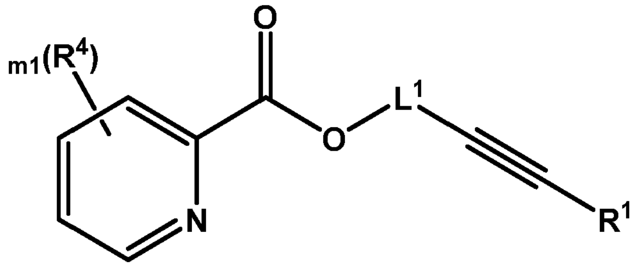

Chemical Formula 1J

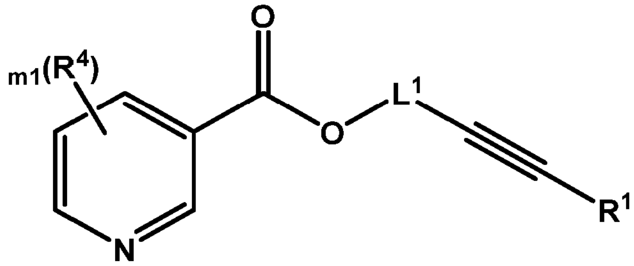

Chemical Formula 1K

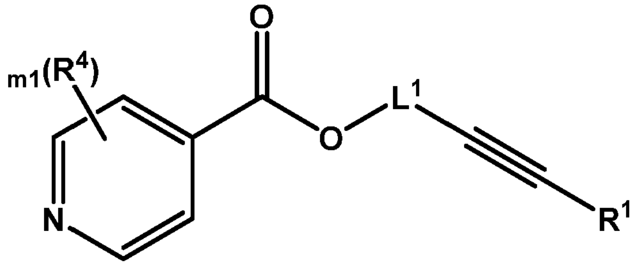

Chemical Formula 1L

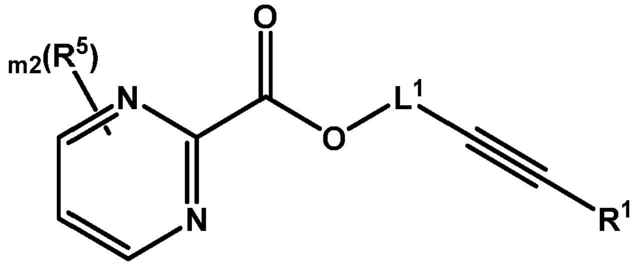

Chemical Formula 1M

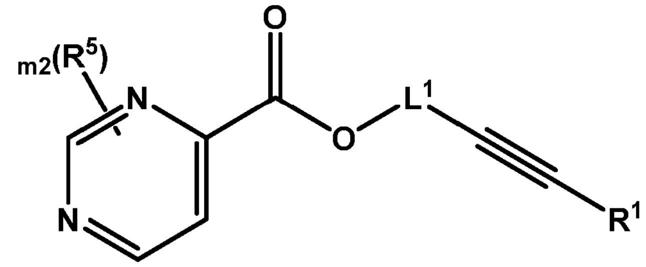

Chemical Formula 1N

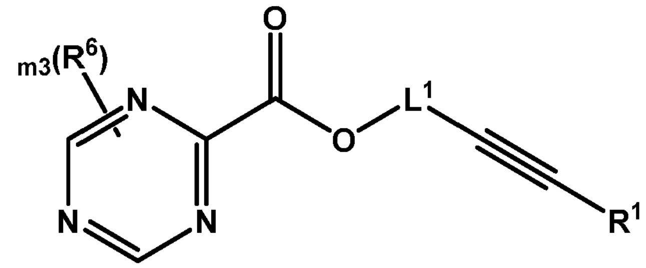

Chemical Formula 1O

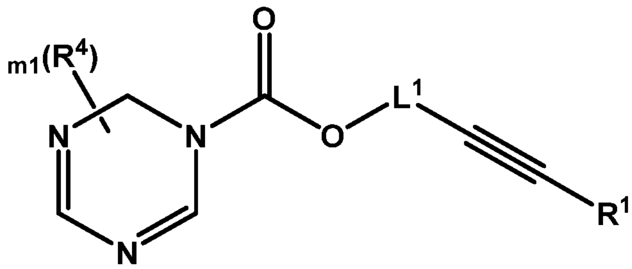

,

and
wherein, in Chemical Formula 1A to Chemical Formula 1O,

$L^1$ is a substituted or unsubstituted C1 to C3 alkylene group,
$R^1$ and $R^4$ to $R^8$ are each independently hydrogen or a substituted or unsubstituted C1 to C10 alkyl group,
m1 is one of integers 1 to 4,
m2 is one of integers 1 to 3,
m3 is an integer of 1 or 2, and
m4 is one of integers 1 to 6.

**9.** The rechargeable lithium battery of claim 1, wherein
the compound represented by Chemical Formula 1 is selected from among compounds listed in Group 1:

Group 1

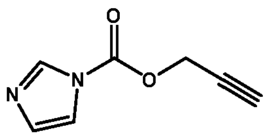 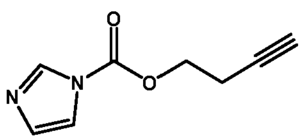 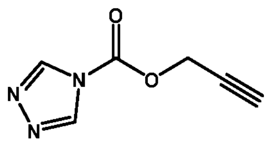 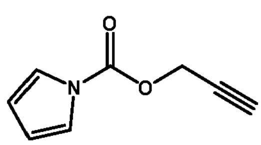

.

**10.** The rechargeable lithium battery of any one of the preceding claims, wherein
the compound represented by Chemical Formula 1 is comprised in an amount of 0.05 to 5.0 parts by weight based on

100 parts by weight based on 100 parts by weight of the total electrolyte solution (lithium salt + non-aqueous organic solvent) except the additive.

**11.** The rechargeable lithium battery of any one of the preceding claims, wherein the electrolyte solution further comprises at least one of other additives selected from among vinylene carbonate (VC), fluoroethylene carbonate (FEC), difluoroethylene carbonate, chloroethylene carbonate, dichloroethylene carbonate, bromoethylene carbonate, dibromoethylene carbonate, nitroethylene carbonate, cyanoethylene carbonate, vinylethylene carbonate (VEC), adiponitrile (AN), succinonitrile (SN), 1,3,6-hexane tricyanide (HTCN), propensultone (PST), propanesultone (PS), lithium tetrafluoroborate ($LiBF_4$), lithium difluorophosphate ($LiPO_2F_2$), and 2-fluorobiphenyl (2-FBP).

**12.** The rechargeable lithium battery of any one of the preceding claims, wherein

the lithium nickel manganese-based oxide comprises a cobalt-free lithium composite oxide represented by Chemical Formula 4:

Chemical Formula 4 $Li_aNi_xMn_yM^1_zM^2_wO_{2\pm b}X_c$, and

wherein, in Chemical Formula 4,
$0.5 \leq a < 1.8$, $0 \leq b \leq 0.1$, $0 \leq c \leq 0.1$, $0 \leq w < 0.1$, $0.6 \leq x < 1.0$, $0 < y < 0.4$, $0 < z < 0.1$, $w + x + y + z = 1$,

$M^1$ and $M^2$ are each independently at least one element selected from among Al, Mg, Ti, Zr, Cr, Sr, V, B, W, Mo, Si, Ba, Ca, Ce, Fe, and Nb, and
X is at least one element selected from among S, F, P, and Cl.

**13.** The rechargeable lithium battery of claim 12, wherein

Chemical Formula 4 is represented by Chemical Formula 4-1:

Chemical Formula 4-1 $Li_aNi_{x1}Mn_{y1}Al_{z1}M^2_{w1}O_{2\pm b}X_c$

, and
wherein, in Chemical Formula 4-1,
$0.5 \leq a< 1.8$, $0 \leq b \leq 0.1$, $0 \leq c \leq 0.1$,$0 \leq w1 <0.1$, $0.6 \leq x1 < 1.0$, $0 < y1 <0.4$, $0 < z1 < 0.1$, $w1 + x1 + y1 + z1 = 1$,

each $M^2$ is independently at least one element selected from among Mg, Ti, Zr, Cr, Sr, V, B, W, Mo, Si, Ba, Ca, Ce, Fe, and Nb, and
X is at least one element selected from among S, F, P, and Cl.

**14.** The rechargeable lithium battery of claim 13, wherein in Chemical Formula 4-1, x1 is $0.6 \leq x1 \leq 0.79$, y1 is $0.2 \leq y1 \leq 0.39$, and z1 is $0.01 \leq z1 < 0.1$.

**15.** The rechargeable lithium battery of any one of the preceding claims, wherein the negative electrode active material comprises at least one of graphite and Si composite.

FIG. 1

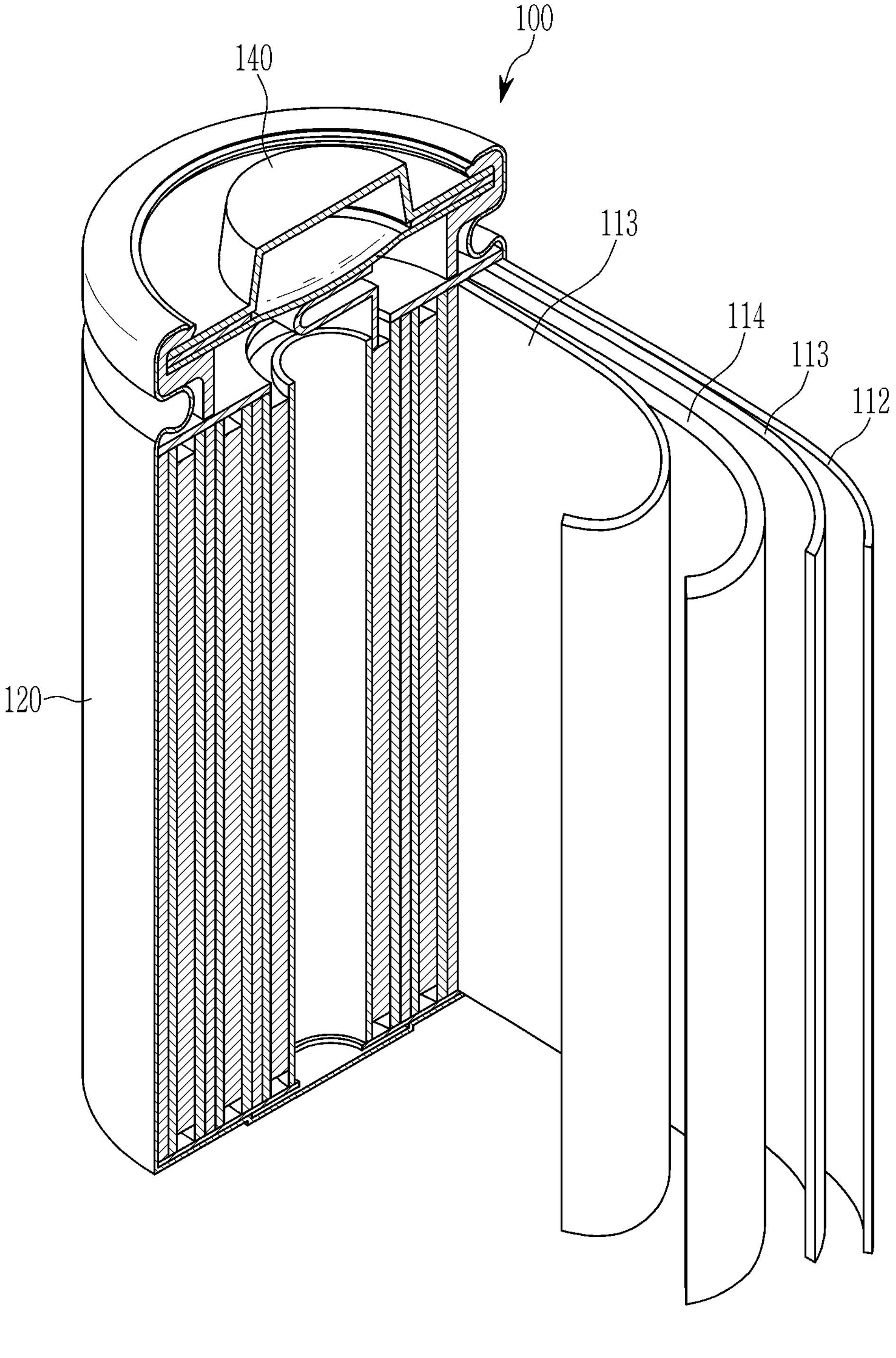

Europäisches Patentamt
European Patent Office
Office européen des brevets

# EUROPEAN SEARCH REPORT

Application Number
EP 24 16 5472

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2022/115701 A1 (AN YU HA [KR] ET AL) 14 April 2022 (2022-04-14)<br>* paragraphs [0037] - [0040], [0044], [0078] - [0084], [0089] - [0094], [0106] *<br>* example 1 *<br>----- | 1-15 | INV.<br>H01M4/62<br>H01M10/0567<br>H01M4/505<br>H01M4/525<br>H01M10/0569 |
| X | US 2020/044287 A1 (KIM HYUN SEUNG [KR] ET AL) 6 February 2020 (2020-02-06)<br>* paragraphs [0027] - [0030], [0048] - [0056], [0109] - [0111], [0115], [0124] - [0127] *<br>----- | 1-3,5-15 | |
| A | US 2019/089003 A1 (NOGUCHI TAKEHIRO [JP] ET AL) 21 March 2019 (2019-03-21)<br>* examples 7, 8; table 1 *<br>----- | 1-15 | |
| A | US 2022/131195 A1 (JI LIWEN [US] ET AL) 28 April 2022 (2022-04-28)<br>* paragraph [0069] *<br>----- | 1-15 | TECHNICAL FIELDS SEARCHED (IPC) |
| A | ZHENG XI ET AL: "Exploring high-voltage fluorinated carbonate electrolytes for LiNi0.5Mn1.5O4 cathode in Li-ion batteries",<br>JOURNAL OF ENERGY CHEMISTRY, ELSEVIER, AMSTERDAM, NL,<br>vol. 42, 4 June 2019 (2019-06-04), pages 62-70, XP085914418,<br>ISSN: 2095-4956, DOI: 10.1016/J.JECHEM.2019.05.023<br>[retrieved on 2019-06-04]<br>* 2.2 Electrolyte preparation and characterization;<br>page 63 *<br>----- | 1-15 | H01M |

The present search report has been drawn up for all claims

| Place of search | Date of completion of the search | Examiner |
|---|---|---|
| The Hague | 11 September 2024 | Forestier, Gilles |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

4

EPO FORM 1503 03.82 (P04C01)

# ANNEX TO THE EUROPEAN SEARCH REPORT ON EUROPEAN PATENT APPLICATION NO.

EP 24 16 5472

This annex lists the patent family members relating to the patent documents cited in the above-mentioned European search report.
The members are as contained in the European Patent Office EDP file on
The European Patent Office is in no way liable for these particulars which are merely given for the purpose of information.

11-09-2024

| Patent document cited in search report | Publication date | Patent family member(s) | Publication date |
|---|---|---|---|
| US 2022115701 A1 | 14-04-2022 | EP 3902051 A1 | 27-10-2021 |
| | | JP 7154677 B2 | 18-10-2022 |
| | | JP 2022517684 A | 09-03-2022 |
| | | US 2022115701 A1 | 14-04-2022 |
| | | WO 2020153791 A1 | 30-07-2020 |
| US 2020044287 A1 | 06-02-2020 | CN 110574210 A | 13-12-2019 |
| | | EP 3518334 A1 | 31-07-2019 |
| | | KR 20190008100 A | 23-01-2019 |
| | | PL 3518334 T3 | 23-08-2021 |
| | | US 2020044287 A1 | 06-02-2020 |
| US 2019089003 A1 | 21-03-2019 | CN 108780922 A | 09-11-2018 |
| | | JP 6977708 B2 | 08-12-2021 |
| | | JP WO2017154788 A1 | 17-01-2019 |
| | | US 2019089003 A1 | 21-03-2019 |
| | | WO 2017154788 A1 | 14-09-2017 |
| US 2022131195 A1 | 28-04-2022 | US 2022131195 A1 | 28-04-2022 |
| | | WO 2022093792 A1 | 05-05-2022 |

EPO FORM P0459

For more details about this annex : see Official Journal of the European Patent Office, No. 12/82